**Europäisches Patentamt**

**European Patent Office**

**Office européen des brevets**

(19)

(11) Veröffentlichungsnummer: **0 001 383**
**B1**

# (12) EUROPÄISCHE PATENTSCHRIFT

(45) Veröffentlichungstag der Patentschrift:
**12.08.81**

(51) Int. Cl.³: **C 07 D 333/54**

(21) Anmeldenummer: **78100329.8**

(22) Anmeldetag: **07.07.78**

(54) **Verfahren zur Herstellung von Thionaphthen aus Thionaphthensulfonsäure.**

(30) Priorität: **30.09.77 DE 2744066**

(43) Veröffentlichungstag der Anmeldung:
**18.04.79 Patentblatt 79/8**

(45) Bekanntmachung des Hinweises auf die Patenterteilung:
**12.08.81 Patentblatt 81/32**

(84) Benannte Vertragsstaaten:
**BE DE FR GB NL**

(56) Entgegenhaltungen:
**DE-A-2 535 192**
**DE-C-325 712**
**DE-C-333 156**

(73) Patentinhaber: **Rütgerswerke Aktiengesellschaft,**
**Mainzer Landstrasse 217, D-6000 Frankfurt a.Main 1 (DE)**

(72) Erfinder: **Grigoleit, Georg, Dr., Moselweg 7,**
**D-4270 Dorsten 19 (DE)**
Erfinder: **Matern, Kurt, Quadtstrasse 9,**
**D-4100 Duisburg 12 (DE)**
Erfinder: **Köhler, Helmut, Dr., Mülheimer Strasse 208,**
**D-4100 Duisburg (DE)**
Erfinder: **Collin, Gerd, Dr., Hagensallee 56,**
**D-4100 Duisburg 12 (DE)**

Verfahren zur Herstellung von Thionaphthen aus Thionaphthensulfonsäure

Die vorliegende Erfindung betrifft ein Verfahren zur Herstellung von Thionaphthen durch Spaltung von Thionaphthensulfonsäure, wie sie z. B. bei der partiellen Sulfonierung von Naphthalinfraktionen des Steinkohlenteers anfällt (vgl. Franck u. Collin, Steinkohlenteer, Springer-Verlag, 1968, Seite 72).

Aus der DE-AS 25 35 192 ist es bekannt, Thionaphthen aus entsprechenden Teerölfraktionen durch partielle Sulfonierung und anschließende Spaltung des entstandenen Sulfonsäuregemisches mit überhitztem Wasserdampf zu isolieren.

Thionaphthen, das nach diesem Verfahren bei Temperaturen von ca. 115 bis 140°C in etwa 90%iger Anreicherung anfällt, wird in einem weiteren Reinigungsschritt durch Destillation und Kristallisation in reiner Form gewonnen.

Für eine technische Gewinnung von Thionaphthen ist dieses Verfahren aber wenig geeignet, da der Spaltprozeß äußerst langwierig ist und damit zu erheblichen Kosten führt. Dadurch konnten die Einsatzgebiete für Thionaphthen und seine Derivate (vgl. Franck u. Collin, l. c., Seite 183) bisher keine praktische Bedeutung erlangen.

Aufgabe der Erfindung war es daher, ein neues Verfahren zur Gewinnung von Thionaphthen durch Spaltung der Thionaphthensulfonsäure zu finden, das in industriellem Maßstab durchgeführt werden kann und das auf einer einfachen und billigen Methode beruht. Diese Aufgabe wird durch das erfindungsgemäße Verfahren gelöst.

Gegenstand der Erfindung ist ein Verfahren zur Herstellung von Thionaphthen aus Thionaphthensulfonsäure durch Spaltung mit Wasser bei 120–180°C, dadurch gekennzeichnet, daß während der gesamten Reaktionsdauer die im Bereich von $1,07 \cdot 10^3$ bis $1,4 \cdot 10^3$ kg/m$^3$ liegende Dichte des wäßrigen Reaktionsgemisches durch Zugabe von Wasser konstant gehalten und das sich bildende Thionaphthen nicht entfernt wird.

Im Gegensatz zu den bisher üblichen Methoden der Spaltung mit überhitztem Wasserdampf unter gleichzeitiger Wasserdampf-Destillation des anfallenden Thionaphthens wurde gefunden, daß nach dem erfindungsgemäßen Verfahren bei zumindest gleichen Ausbeuten wesentlich kürzere Reaktionszeiten ausreichen, wodurch ein in industriellem Maßstab einfach durchzuführendes Verfahren geschaffen wurde. Bei Betriebsansätzen hat es sich gezeigt, daß sich bei der herkömmlichen Wasserdampf-Spaltung durchschnittlich z. B. nur etwa 15 kg Thionaphthen mit etwa der zehnfachen Wassermenge durch Wasserdampfdestillation stündlich abtrennen lassen. Das bedeutet, daß man für die Sulfonsäurespaltung und Destillation zur Gewinnung von einer Tonne Thionaphthen etwa 70 bis 80 Stunden aufwenden müßte. Nach dem erfindungsgemäßen Verfahren hingegen kann die gleiche Menge in wesentlich kürzerer Zeit (ca. 10 Stunden) erhalten werden, da nach diesem Verfahren die langwierige Wasserdampf-Destillation entfällt.

Das erfindungsgemäße Verfahren wird insbesondere zur Gewinnung von Thionaphthen aus technischer Thionaphthensulfonsäure, wie sie nach der partiellen Sulfonierung von Rohnaphthalin aus Steinkohlenteer (z. B. nach der DE-AS 25 35 192) erhalten werden kann, eingesetzt. Dadurch besteht die Möglichkeit, neben reinstem Naphthalin auch das Thionaphthen auf einfache und industriell verwertbare Weise zu erhalten. Unter »Reaktionsgemisch« wird eine wäßrige Thionaphthensulfonsäurelösung verstanden, wie sie z. B. bei der Aufarbeitung der partiellen Sulfonierung von Rohnaphthalin erhalten wird (vgl. DE-AS 25 35 192).

Das Verfahren wird im Dichtebereich von $1,07 \cdot 10^3$ bis etwa $1,40 \cdot 10^3$ kg/m$^3$ und bei Temperaturen von 120 bis 180°C durchgeführt. Dabei entsprechen höheren Dichten vorzugsweise niedrigere Reaktionstemperaturen und umgekehrt.

Der genannte Dichtebereich entspricht dabei Konzentrationen, wie sie normalerweise in den durch partielle Sulfonierung von Rohnaphthalin erhaltenen Sulfonierungsgemischen vorliegen oder daraus durch Einengen erhalten werden können.

Das erfindungsgemäße Verfahren kann entweder diskontinuierlich (ansatzweise) oder kontinuierlich durchgeführt werden. Arbeitet man diskontinuierlich, so wird vorzugsweise bei Dichten von $1,30 \cdot 10^3$ bis $1,40 \cdot 10^3$ kg/m$^3$, insbesondere $1,30 \cdot 10^3$ bis $1,33 \cdot 10^3$ kg/m$^3$ unter Rückfluß gearbeitet, wodurch sich Temperaturen von ca. 120–130°C einstellen, während bei kontinuierlicher Arbeitsweise bei Dichten von ca. $1,22 \cdot 10^3$ bis $1,07 \cdot 10^3$ kg/m$^3$ bei Temperaturen zwischen 150 und 180°C, insbesondere 155 bis 160°C, in einem Dünnschichtreaktor gearbeitet wird.

Durch kontrollierte Zugabe von Wasser läßt sich die ursprüngliche Dichte der Reaktionsmischung im wesentlichen konstant halten, wobei Abweichungen der Dichtewerte von bis zu $\pm 0,04 \cdot 10^3$ das Ergebnis noch nicht wesentlich beeinflussen.

## Beispiel 1

In einen Reaktionskolben, versehen mit Rührwerk, Einleitungsrohr, Thermometer und Rückflußkühler mit Destillationsabgang, werden 100 Teile einer technischen Thionaphthensulfonsäure, Dichte $1,33 \cdot 10^3$ kg/m$^3$, wie sie nach dem Verfahren der DE-AS 25 35 192 durch Einengen gewonnen werden kann, vorgelegt.

**0 001 383**

Nach Aufheizung auf 120° werden innerhalb von 90 Minuten unter Rühren 50 Teile kaltes Wasser zugespeist. Während des exothermen Spaltprozesses werden 30 Teile Wasser aus dem Reaktionskolben verdampft und kondensiert. Am Ende der Säurespaltung hat sich eine Temperatur von 125° eingestellt. Die Enddichte der Reaktionsmischung beträgt $1{,}37 \cdot 10^3$ kg/m³. Nach Abkühlung auf etwa 30° wird die ölige Phase über ein Scheidegefäß abgezogen und mit Wasser und verdünnter Lauge neutralisiert. In einer Reinheit von 85% werden 83% d. Th. an Thionaphthen vom Erstarrungspunkt 24°C erhalten. Durch Destillation und Kristallisation wird ein 98- bis 99%iges Thionaphthen gewonnen.

Beispiel 2

In einen vertikal installierten Dünnschichtreaktor, versehen mit einem beheizten Vorwärmeteil, Wischerblattrotor und Kondensationsvorlage mit Scheidegefäß werden mittels einer Dosiereinrichtung bei Manteltemperaturen des Reaktors von 155 bis 160° kontinuierlich technische Thionaphthensulfonsäure, wie sie nach dem Verfahren der DE-AS 25 35 192 anfällt, eindosiert. Die Sulfonsäure hat eine Dichte von $1{,}07 \cdot 10^3$ kg/m³. Die Flüssigkeitsraumgeschwindigkeit beträgt $0{,}7$ h$^{-1}$.

Die Spaltung der Sulfonsäure setzt nach Eintritt in den Reaktor fast augenblicklich ein, was sich durch Schlierenbildung an der Glaswand des Reaktors anzeigt. Nach Verlassen des Reaktors wird das gespaltene Material über ein Scheidegefäß getrennt. Die untere Thionaphthenphase wird in einer Extraktionskolonne kontinuierlich mit verdünnter Lauge neutralisiert. Die Enddichte der Reaktionsmischung beträgt $1{,}06 \cdot 10^3$ kg/m³. In einer Ausbeute von 92% d. Th. wird ein 78%iges Thionaphthen erhalten (Erstarrungspunkt 20°), das durch Destillation und Kristallisation auf eine Reinheit von 98% gebracht wird.

Analog Beispiel 1 oder 2 erhält man bei Änderung der Reaktionsbedingungen die in der folgenden Tabelle zusammengestellten Ergebnisse.

RF  bedeutet Spaltung unter Rückfluß,
DS  Spaltung im Dünnschichtreaktor und
WD die zum Vergleich herangezogene Wasserdampfspaltung bei gleichzeitiger Wasserdampf-Destillation.

3

| | Ausgangsmaterial | $D_4^{20}$ [kg/m³] | Spaltbedingungen | | Thionaphthen |
|---|---|---|---|---|---|
| | | | Temp. [°C] | Zeit in Min. bzw. Flüssigkeitsraum-geschwindigkeit | Ausbeute [%] |
| RF | Thionaphthen-sulfonsäure-(2) rein | $1,33 \cdot 10^3$ | 120 | 180 | 80,0 |
| | | $1,33 \cdot 10^3$ | 120 | 180 | 81,8 |
| | | $1,33 \cdot 10^3$ | 120 | 180 | 82,5 |
| | | $1,33 \cdot 10^3$ | 125 | 60 | 71,1 |
| DS | Thionaphthen-sulfonsäure-(2) rein | $1,22 \cdot 10^3$ | 160 | 0,7 | 81,5 |
| | | $1,15 \cdot 10^3$ | 160 | 0,7 | 85,2 |
| | | $1,15 \cdot 10^3$ | 155 | 0,7 | 86,1 |
| | | $1,15 \cdot 10^3$ | 180 | 0,7 | 75,1 |
| | | $1,07 \cdot 10^3$ | 155 | 0,7 | 79,8 |
| WD | Thionaphthen-sulfonsäure-(2) rein | $1,33 \cdot 10^{3*})$ | 130 | 435 | 81,9 |
| WD | Thionaphthen-sulfonsäure techn. | $1,33 \cdot 10^{3*})$ | 130 | 480 | 83,8 |
| RF | | $1,33 \cdot 10^3$ | 125 | 90/240 | 83,3 |
| DS | Thionaphthen-sulfonsäure techn. | $1,07 \cdot 10^3$ | 155 | 0,7 | 92,1 |
| | | $1,07 \cdot 10^3$ | 155 | 0,7 | 91,6 |
| | | $1,07 \cdot 10^3$ | 160 | 0,7 | 91,8 |
| | | $1,07 \cdot 10^3$ | 180 | 0,7 | 72,9 |

*) Anfangswerte.

## Patentansprüche

1. Verfahren zur Herstellung von Thionaphthen aus Thionaphthensulfonsäure durch Spaltung mit Wasser bei 120 – 180°C, dadurch gekennzeichnet, daß während der gesamten Reaktionsdauer die im Bereich von $1,07 \cdot 10^3$ bis $1,4 \cdot 10^3$ kg/m³ liegende Dichte des wäßrigen Reaktionsgemisches durch Zugabe von Wasser konstant gehalten und das sich bildende Thionaphthen nicht entfernt wird.

2. Verfahren nach Anspruch 1, dadurch gekennzeichnet, daß man bei einer Dichte des Reaktionsgemisches im Bereich von $1,30 \cdot 10^3$ bis $1,4 \cdot 10^3$ kg/m³ und einer Temperatur im Bereich von 120 – 130°C arbeitet.

3. Verfahren nach Patentanspruch 1, dadurch gekennzeichnet, daß man bei einer Dichte des Reaktionsgemisches im Bereich von $1,07 \cdot 10^3 – 1,22 \cdot 10^3$ kg/m³ und einer Temperatur im Bereich von 150 – 180°C arbeitet und die Reaktion in einem Dünnschichtreaktor durchführt.

## Claims

1. A process for the production of thionaphthene by dissociation of thionaphthene sulfonic acid with water at a temperature of 120 – 180°C, characterized in that during the entire duration of the reaction the density of the aqueous reaction mixture, which is in the range of $1,07 \cdot 10^3$ to $1,4 \cdot 10^3$ kg/m$^3$ is maintained constant by addition of water and that the thionaphthene formed is not removed.

2. The process according to claim 1, characterized in that the reaction is conducted at a density of the reaction mixture in the range of $1,30 \cdot 10^3$ to $1,40 \cdot 10^3$ kg/m$^3$ and at a temperature in the range of $120-130°$C.

3. The process according to claim 1, characterized in that the reaction is conducted at a density of the reaction mixture in the range of $1,07 \cdot 10^3$ to $1,22 \cdot 10^3$ kg/m$^3$ and at a temperature in the range of 150 to 180° C in a film reactor.

**Revendications**

1. Procédé d'obtention de thionaphthène à partir d'acide thionaphthènesulfonique par coupure par l'eau à $120-180°$C, caractérisé par le fait que pendant toute la durée réactionnelle, la densité du mélange réactionnel aqueux se trouvant dans le domaine de $1,07 \cdot 10^3$ à $1,4 \cdot 10^3$ kg/m$^3$ est maintenue constante par addition d'eau et que le thionaphthène qui se forme n'est pas enlevé.

2. Procédé selon la revendication 1, caractérisé par le fait que l'on opère avec une densité du mélange réactionnel dans un domaine de $1,30 \cdot 10^3$ à $1,4 \cdot 10^3$ kg/m$^3$ et une température dans un domaine de $120-130°$C.

3. Procédé selon la revendication 1, caractérisé par le fait que l'on opère avec une densité du mélange réactionnel dans le domaine de $1,07 \cdot 10^3$ à $1,22 \cdot 10^3$ kg/m$^3$ et une température dans le domaine de $150-180°$C et que l'on réalise la réaction dans un réacteur à couche mince.